(19) Europäisches Patentamt
European Patent Office
Office européen des brevets

(11) Numéro de publication : **0 504 031 A1**

# (12) DEMANDE DE BREVET EUROPEEN

(21) Numéro de dépôt : **92400608.3**

(22) Date de dépôt : **09.03.92**

(51) Int. Cl.$^5$ : **G06F 15/60,** C07K 3/00

(30) Priorité : **11.03.91 FR 9102911**

(43) Date de publication de la demande :
**16.09.92 Bulletin 92/38**

(84) Etats contractants désignés :
**DE FR GB IT NL**

(71) Demandeur : **COMMISSARIAT A L'ENERGIE ATOMIQUE**
**31-33, rue de la Fédération**
**F-75015 Paris (FR)**

(72) Inventeur : **Niel, Jean-Christophe**
**82 rue de la République**
**F-92190 Meudon-la-Foret (FR)**
Inventeur : **Orland, Henri**
**15-17 rue Cels**
**F-75014 Paris (FR)**
Inventeur : **Garel, Thomas**
**15 Allée Elisabeth de Feydeau**
**F-91190 Gif-sur-Yvette (FR)**

(74) Mandataire : **Mongrédien, André et al**
**c/o BREVATOME 25, rue de Ponthieu**
**F-75008 Paris (FR)**

## (54) Procédé et dispositif de traitement pour l'obtention des conformations tridimensionnelles d'une macromolécule à l'équilibre thermodynamique à partir de sa structure chimique.

(57) Procédé et dispositif de traitement pour l'obtention des conformations tridimensionnelles d'une macromolécule à l'équilibre thermodynamique à partir de sa structure chimique.

En partant de la structure d'une molécule, des énergies d'interaction entre ses atomes, ce procédé consiste à réaliser, à une température fixée par un thermostat (T), un ensemble statistique de Boltzmann de configurations de coordonnées de ces atomes en faisant croître dans des moyens de traitement (UC) une population de chaînes d'atomes, atome par atome ou groupe d'atomes par groupe d'atomes, en calculant l'énergie partielle des chaînes à chaque ajout d'un atome ou groupe d'atomes, en calculant le poids de Boltzmann relatif de chaque configuration issue de la croissance, en répliquant ou supprimant chaque configuration issue de la croissance proportionnellement au poids de Boltzmann calculé, à minimiser avec des moyens de minimisation (S) l'énergie des configurations dudit ensemble, judicieusement choisies et à visualiser les configurations minimisées choisies sur des moyens de visualisation (V), ces dernières constituant les conformations spatiales recherchées.

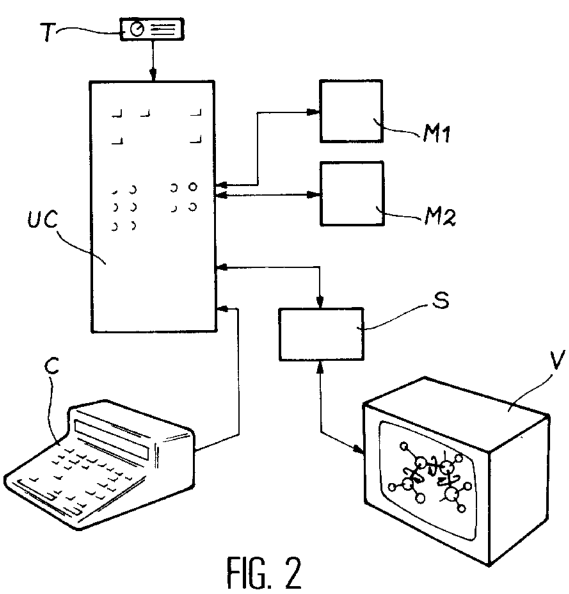


FIG. 2

EP 0 504 031 A1

L'invention se rapporte à un procédé d'obtention des conformations spatiales d'une macromolécule, à une température donnée, à partir de sa structure chimique ainsi qu'un dispositif de traitement pour la mise en oeuvre de ce procédé. Elle s'applique dans les domaines de l'industrie chimique, biologique et pharmacologique et du génie génétique.

Le procédé de l'invention peut s'appliquer à toute molécule organique ou minérale et plus spécialement à toute macromolécule comportant au moins 12 atomes comme par exemple les protéines (hormones, anticorps, enzymes), les polypeptides, les acides nucléiques (ARN, ADN), les polymères.

Les macromolécules que l'on rencontre en chimie, pharmacie et biologie (en particulier les protéines) sont formées d'une chaîne principale sur laquelle se greffent des chaînes latérales.

On peut ainsi représenter la structure générale (I) d'une telle macromolécule par une chaîne principale et des résidus notés $R_1$, $R_2$, ...

(I) 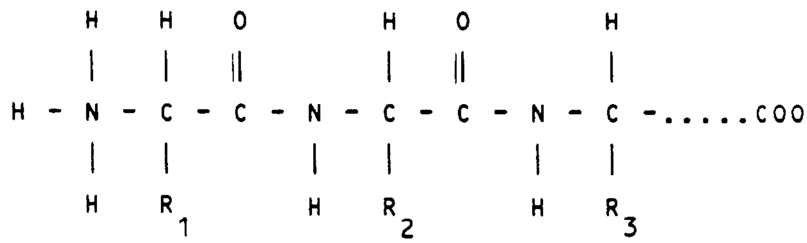

Les résidus $R_1$, $R_2$ (chaînes latérales) peuvent être choisis parmi 20 acides aminés dont certains sont représentés ci-après :

$-CH_2SH$ Cystéine

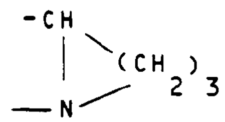

Prodine

$-CH_3$ alanine

$-CH_2-C_6H_5$ phénylalanine

Les liaisons en traits pleins entre espèces chimiques représentent les liaisons covalentes. Ces liaisons ne sont pas rigides mais assimilées à des "ressorts" de forte raideur dans l'échelle d'unités adéquates.

Dans la suite de la description, on utilisera le terme générique de macromolécules pour désigner l'ensemble des hétéropolymères, acides nucléiques, polypeptides ou toute autre molécule chimique.

Un des problèmes majeurs de l'étude des macromolécules est de prédire leurs conformations spatiales de basse énergie ; par conformations il faut comprendre la position spatiale des N atomes constituant la macromolécule. L'ensemble de ces 3N coordonnées décrit l'espace de phases du système, c'est-à-dire l'espace des degrés de liberté dynamiques.

(Ces conformations de basse énergie peuvent être comparées aux structures présentes dans le cristal de la protéine considérée - à condition de savoir la cristalliser -, aux résultats issus de la Résonance Magnétique Nucléaire (R.M.N.) etc.).

L'enjeu biologique de la détermination de ces structures de basse énergie est considérable, puisque la fonction biologique de ces macromolécules est très intimement liée à ces structures.

La complexité de ce problème à grand nombre de variables (dès que le nombre d'atomes constituant N de la macromolécule est grand - plusieurs milliers d'atomes -) est accrue par le fait que le milieu ambiant usuel de ces protéines est constitué d'eau.

Tous les procédés d'obtention des conformères de basse énergie d'une macromolécule existants (y compris celui objet de l'invention) supposent qu'il existe une énergie d'interaction phénoménologique E qui dépend d'une part des positions des atomes de la macromolécule (c'est-à-dire de sa géométrie) et d'autre part de paramètres fixés qui peuvent être déduits de l'expérience ou de certaines banques de données. Dans ces procédés, on se place essentiellement dans le vide où la constante diélectrique vaut l'unité. Toutefois, la généralisation à un milieu ambiant aqueux pourra être faite.

Dans tous les procédés connus, et y compris dans celui de l'invention, on cherche à atteindre les configurations de basse énergie d'une macromolécule à l'équilibre thermodynamique ; on considère donc un système couplé à un thermostat permettant de définir une température T. Tous ces procédés se composent de deux étapes :

A) - Une étape d'échantillonnage de l'espace des phases du système, et

B) - Une étape de minimisation de l'énergie des configurations obtenues.

L'étape B consiste à rechercher localement un minimum de l'énergie par petites variations des positions des atomes. L'étape A est au contraire cruciale pour atteindre le minimum absolu et c'est dans celle-ci que le phénomène de frustration existe et peut conduire à l'obtention d'états métastables qui sont des minima locaux de l'énergie E et non pas le minimum absolu.

Ceci est représenté sur la figure 1 annexée illustrant les variations de l'énergie E de la molécule en fonction des coordonnées des atomes de la molécule.

Dans la figure 1 annexée, le minimum recherché est en M, toutefois, la minimisation (étape B) à partir d'une configuration de coordonnées qui serait initialement en 1, 2 ou 3 conduirait à I, J ou K. Par contre, une minimisation à partir de 4 ou 5 pourraît aboutir en M. En effet, le processus de minimisation permet éventuellement le franchissement de petites barrières en énergie comme par exemple 5 convergeant vers le point M, mais ne permet pas le franchissement de grandes barrières, comme de 1 vers M.

A l'issue de l'étape A, les configurations obtenues doivent être convenablement réparties "au-dessus du paysage en énergie". Aussi, dans le cas de la figure, il faut qu'il existe des configurations de coordonnées de type 4 ou 5.

Les procédés connus d'obtention des conformères d'une macromolécule utilisent soit une technique de dynamique moléculaire, soit une technique de Monte Carlo ou soit une technique de recuit simulé, pour échantillonner l'espace des phases du système.

Dans la technique de dynamique moléculaire pour l'échantillonnage des phases :

i) - on part d'une configuration initiale de la macromolécule (par exemple choisie au hasard),

ii) - on essaie d'atteindre l'équilibre thermodynamique à température T, en injectant de l'énergie au système en alternance avec une évolution suivant les lois de la dynamique classique. Cette température T n'est pas nécessairement physique, elle sert à échantillonner,

iii) - on laisse le système évoluer, à l'équilibre thermodynamique, suivant les lois de la dynamique classique, soit :

$$m_i \ddot{\vec{r_i}} = - \frac{\delta E}{\delta \vec{r_i}}$$

si $\vec{r_i}$ est le vecteur position de l'atome i (noté de 1 à N), mi sa masse et E l'énergie du système. Cette évolution continue alors pendant un temps donné (typiquement 100 ps).

Il existe une variante de ce procédé basée sur l'équation de Langevin où l'énergie injectée (étape ii) l'est sous forme d'une force de frottement aléatoire.

L'inconvénient essentiel de la dynamique moléculaire est que le système conserve souvent la mémoire de la configuration initiale (étape i) en particulier dans les cas où le profil en énergie de l'espace des phases possède de hautes barrières séparant pratiquement celui-ci en régions indépendantes.

Ainsi, si l'étape (ii) n'est pas achevée (équilibre non atteint) ou si l'étape (iii) n'est pas assez longue alors le processus de minimisation qui suit l'échantillonnage va aboutir à de faux minima.

Dans les techniques de Monte Carlo traditionnelles, on considère encore les macromolécules complètes. A partir d'une conformation définie par les positions spatiales des N atomes de la molécule, on bouge l'atome i choisi de manière aléatoire ou séquentielle d'une quantité $\delta\vec{r_i}$. Ce mouvement s'accompagne d'une variation d'énergie δE de l'énergie E de la macromolécule.

– si $\delta E<0$, le mouvement de l'atome i est accepté et on recommence l'opération avec un autre atomes j de la macromolécule que l'on bouge de $\delta\vec{r_j}$

– si $\delta E>0$, le mouvement de l'atome i est accepté avec la probabilité $p=\exp(-\beta\delta E)$où $\beta^{-1}=kT$, k étant la constante de Boltzmann et T la température (non nécessairement physique) à laquelle on veut échantillonner le système ; puis on choisit un autre atome j que l'on bouge de $\delta\vec{r_j}$.

Le balayage de la macromolécule peut être aléatoire ou séquentiel ; il est clair que le choix de la température T est primordial : si T est petit, alors seules peuvent être franchies les barrières d'énergie d'ordre $\Delta E<kT$, le système restant piégé autour du minimum local le plus proche, si T est trop grand, l'espace de phases à échantillonner est trop large et le procédé n'est pas efficace.

Dans le cas des protéines, les énergies covalentes sont très couteuses en énergie et vont donc interdire les déplacements entraînant une variation des longueurs des liaisons covalentes trop importante (il en est de même pour les angles de valence).

Dans la technique de recuit simulé, on essaie d'éviter de tomber dans les minima locaux. Elle peut être décrite comme suit :

i') - à l'aide d'une méthode de Monte Carlo traditionnelle, telle que décrite ci-dessus, on évolue autour d'un minimum local A,

ii') - on réchauffe le système d'une quantité $\Delta T$, ce qui permet d'échantillonner une plus grande portion de l'espace des phases. On peut donc franchir des barrières d'énergie $k(T+\Delta T)$.

iii') - on refroidit lentement le système jusqu'à la température initiale T, c'est-à-dire tout en restant à l'équilibre thermodynamique.

Si le recuit simulé est efficace, il permet de trouver le minimum absolu en partant du minimum relatif A.

Cette méthode a été appliquée aux protéines dans le cas où :

– l'énergie E de la protéine est une énergie d'interaction atome-atome,

– l'énergie E de la protéine est une énergie d'interaction résidu-résidu obtenue à l'aide de banques de données sur les protéines et qui condense toute l'information sur les interactions atome-atome entre deux résidus distincts. Cette énergie d'interaction phénoménologique peut, d'une certaine manière, contenir l'effet de l'eau.

Tous les procédés connus présentent l'inconvénient d'être beaucoup trop longs. En effet, il faut environ 7 heures à ces procédés pour déterminer les conformères d'une molécule comportant 5 acides aminés. Par ailleurs, ces procédés conduisent souvent à l'obtention de minima locaux d'énergie et non au minimum absolu.

La présente invention a justement pour objet un procédé et un dispositif de traitement pour l'obtention des conformations tridimensionnelles d'une macromolécule à l'équilibre thermodynamique à partir de sa structure chimique permettant de remédier à ces inconvénients. En particulier, l'invention permet la détermination des conformères de plus basse énergie d'une molécule de 5 acides aminés en moins de 10 minutes. De plus, l'invention permet d'obtenir un système de conformations spatiales d'une molécule plus proche de la réalité que ceux des procédés connus.

En particulier, le problème de franchissement de barrières en énergie (voir figure) est notoirement plus important pour les procédés traditionnels que pour le procédé de l'invention car ils traitent, dans leur étape A, la molécule dans son ensemble avec ses N atomes (c'est-à-dire avec ses 3N degrés de liberté).

La barrière en énergie (comme celle séparant 1 de 5 dans la figure) doit donc être franchie par les 3N atomes et le piégeage selon l'art antérieur dans des minima locaux est donc relativement facile.

L'invention permet plus spécialement de déterminer la forme repliée des protéines selon un minimum d'énergie à une température donnée.

La différence essentielle entre les procédés connus et le procédé de l'invention concerne la phase A d'échantillonnage de l'espace des phases de la molécule à température donnée T. Le résultat final obtenu à l'issue de cette phase A est que l'ensemble des configurations spatiales de la macromolécule est "boltzmannien", c'est-à-dire qu'il satisfait à la statistique de Boltzmann, et que l'on peut ensuite minimiser ce résultat dans la phase B.

La plupart des procédés connus essayent d'obtenir un ensemble de configurations spatiales "boltzmannien". On rappelle qu'un ensemble de configurations spatiales "boltzmannien" est un ensemble de configurations spatiales où chacune d'entre elles apparaît un nombre de fois égal à son poids de Boltzmann $1/Z(\exp{-\beta E})$ où $\beta^{-1}=kT$ avec k la constante de Boltzmann, T la température d'observation et Z un facteur de normalisation (dénommé fonction de partition).

A titre d'exemple : soit un système pouvant prendre t configurations avec t variant de 1 à P et la configuration t ayant l'énergie Et ; si on considère alors un ensemble de M configurations de ce système, il est "boltzmannien" si la configuration apparant Mt fois avec :

$$Mt = \frac{M\exp(-\beta Et)}{\sum_{u} \exp(-\beta Eu)}$$

Z vaut donc

$$\sum_{u} \exp(-\beta Eu).$$

Dans le procédé de l'invention, on utilise une nouvelle méthode numérique de type Monte Carlo pour obtenir les conformations de basse énergie d'une macromolécule biologique (protéines, acides nucléiques, hétéropolymères) à l'équilibre thermodynamique. Dans ce procédé, les paramètres d'entrée qui doivent être traités sont, d'une part la structure chimique de la molécule, la température d'observation et d'autre part les énergies d'interaction mettant en jeu les différents atomes de la molécule. Ces énergies d'interaction sont d'origine phénoménologique et sont déduites, en principe, de faits expérimentaux ou de bases de données existantes.

De façon plus précise, l'invention concerne un procédé d'obtention des conformations spatiales tridimensionnelles d'énergie les plus basses d'une molécule à l'équilibre thermodynamique, à partir de la structure chimique de cette molécule consistant à :

I) - entrer la structure chimique dans des moyens de mémorisation,

II) - déterminer les énergies d'interaction mettant en jeu les différents atomes de la molécule dans lesdits moyens de mémorisation,

III) - fixer à l'aide d'un thermostat la température à laquelle on veut obtenir lesdites conformations,

IV) - faire croître dans des moyens de traitement une population de chaînes d'atomes, atome par atome ou groupe d'atomes par groupe d'atomes, en calculant l'énergie partielle des chaînes à chaque ajout d'un atome ou d'un groupe d'atomes, en calculant le poids de Boltzmann relatif de chacune des configurations résultant de la croissance, en répliquant ou en supprimant chacune des configurations résultant de la croissance proportionnellement au poids de Boltzmann calculé, afin de réaliser un échantillon statistique de l'ensemble de Boltzmann des configurations de coordonnées des atomes de la molécule,

V) - minimiser l'énergie de configurations dudit échantillon, judicieusement choisies dans les moyens de traitement,

VI) - mémoriser dans des moyens de mémorisation les configurations de coordonnées obtenues en V) constituant les conformations tridimensionnelles d'énergies les plus basses de ladite molécule,

VII) - visualiser les conformations d'énergies les plus basses, obtenus en VI), sur les moyens de visualisation.

L'invention a aussi pour objet un dispositif de traitement pour la mise en oeuvre du procédé ci-dessus. Ce dispositif est défini dans la revendication 1.

Dans l'étape IV, un ensemble de configurations spatiales de coordonnées est engendré de sorte que chacune d'entre elles apparaît un nombre de fois égal à son poids de Boltzmann à la température T. Le poids de Boltzmann d'une configuration est égal à $1/Z(\exp -\beta E)$ où E est l'énergie de la molécule, Z est un facteur de normalisation et $\beta^{-1}=kT$ avec k la constante de Boltzmann et T la température d'observation. Plus l'énergie de la configuration formée est basse, plus sa probabilité d'exister est grande.

Cet ensemble de configurations, dit "boltzmannien", est obtenu par la croissance, atome par atome ou groupe d'atomes par groupe d'atomes, des différentes configurations.

A la suite de chaque étape de la croissance (c'est-à-dire chaque fois que l'on rajoute un atome ou un groupe d'atomes), la molécule partielle ainsi construite est répliquée (reproduite plusieurs fois) ou détruite de manière à favoriser, dans l'ensemble restant, les molécules de plus basse énergie.

Les méthodes de type Monte Carlo usuelles traitent directement la molécule dans son intégralité ; il n'y a donc pas de processus de croissance comme dans le procédé de l'invention.

L'étape V est une étape de minimisation de l'énergie de chaque configuration de cet échantillon "boltzmannien" ou de certaines de ces configurations ; il est possible de la réaliser avec des techniques déjà existantes telles que les techniques de gradient ou de gradients conjugués décrites par exemple dans le document "Monte Carlo minimization approach to the multiple minima problem in protein folding", E. Li et H.A. Scheraga Proceedings of the National Acc. of Sciences USA, vol. 84 (1987), pp. 6611-6615.

De préférence, la minimisation n'est effectuée que sur certaines configurations. Pour ce faire, on réalise avec les moyens de visualisation des tracés de Ramachandran des configurations de l'échantillon statistique comme décrit dans le document "Influence of the local amino-acid sequence upon the zones of the torsional angles $\varphi$ and $\psi$ adopted by residues in proteins", J.F. Jibrat et al., Biochemistry, vol. 30 (1991), pp. 1578-1586, puis on choisit une configuration dans chacun desdits tracés et on minimise l'énergie des configurations ainsi choisies par exemple par la technique de gradients conjugués.

Une manière alternative de décrire le procédé de l'invention est qu'il aboutit à un échantillonnage de l'espace des phases pour les degrés de liberté de la molécule.

La forme de l'énergie E que l'on adopte dans le procédé de l'invention est une énergie entre les divers atomes de la molécule qui portent les variables dynamiques du problème. La forme explicite de E peut être, dans les limites précédentes (vide ou eau), quelconque ; cependant, par référence à la littérature abondante existant déjà sur le sujet, on considère une énergie E qui peut schématiquement s'écrire E = EG + EC + ELJ où :

1°) - EG est une énergie dite géométrique qui dépend des longueurs des liaisons, des angles de valence,

des angles de torsions ; elle agit entre des atomes qui ne sont séparés que par une, deux ou trois liaisons covalentes ; elle est ainsi d'assez courte portée.

1-1 - Les liaisons covalentes.

L'énergie d'interaction associée aux liaisons covalentes est de type harmonique

$$EG^{(1)} = \sum_b Kb(rb - r^\circ b)^2$$

où $\sum_b$ désigne la somme sur les liaisons covalentes, Kb est une constante de raideur, r°b est la longueur de la liaison "au repos" ; les constantes Kb peuvent être déduites de l'analyse de spectres de vibrations infrarouges ; b est par exemple représenté par les atomes formant l'extrémité de la liaison covalente ; b est donc un doublet de chiffre (b=13 pour la liaison entre l'atome numéro 1 et l'atome numéro 3).

1-2 - Les angles de valence.

L'énergie d'interaction associée aux angles de valence est aussi traitée de manière harmonique ainsi :

$$EG^{(2)} = \sum_c Kc(\theta c - \theta^\circ c)^2$$

où $\sum_c$ désigne la somme sur les angles de valences, Kc est une constante de raideur, θ°c est la valeur de l'angle de valence au repos. c est par exemple représenté par un triplet de chiffre dont l'élément central est le sommet de l'angle de valence (exemple c=213, c=657).

1-3 - Les angles dièdres.

Dans la formulation du problème, les angles de torsion s'introduisent naturellement ; ils sont essentiels dans la recherche des conformations de basse énergie de la molécule. On distingue à ce propos les angles de torsion (ou angles dièdres) propres et les angles de torsion (ou angles dièdres) impropres. La définition de ces angles dièdres est liée à des angles entre plans ; elle fait intervenir 4 atomes.

Par exemple, le dièdre propre 1234 est l'angle entre les plans (123) et (234).

L'énergie d'interaction associée aux angles dièdres propres est la suivante :

$$EG^{(3)} = \sum_d Kd(1 + \cos(nd.\psi d - \delta d)),$$

où Kd est une constante de raideur, nd traduit la symmétrie discrète associée à l'angle dièdre (nd vaut 1, 2 ou 3), δd est une phase valant soit 0, soit π en radian, ψd est l'angle des deux plans définis par les 4 atomes du dièdre. En effet, d est par exemple un quadruplet de chiffres (exemple 3157).

Dans le cas des angles dièdres impropres, la valeur de l'énergie d'interaction (de type harmonique) est la suivante :

$$EG^{(4)} = \sum_e Ke(ve - v^\circ e)^2,$$

où Ke est une constante de raideur, v°e la valeur à l'équilibre de l'angle dièdre impropre et ve sa valeur courante ; e est défini par les données des numéros attribués aux 4 atomes composant le dièdre impropre ; e est un quadruplet de chiffres.

On remarque que selon les expressions de l'énergie d'interaction utilisée, les angles dièdres propres {d}

et impropres {e} varient. Par exemple, certaines expressions prennent en compte tous les angles dièdres propres et impropres possibles (c'est-à-dire tous les ensembles de 4 atomes liés entre eux par des liaisons covalentes).

Au total, l'énergie géométrique EG présente dans l'énergie d'interaction totale E de la molécule s'écrit :

$$EG = EG^{(1)} + EG^{(2)} + EG^{(3)} + EG^{(4)}$$
$$= \sum_b Kb(rb-r^ob)^2 + \sum_c Kc(\theta c-\theta^oc)^2 + \sum_d Kd(1+\cos(nd.\phi d-\delta d)) + \sum_e Ke(ve-v^oe)^2.$$

Les divers paramètres (Kb, Kc, Kd, Ke, r°b, θ, nd, δd, v°e) sont déduits (en principe) de l'expérience et sont répertoriés dans des banques de données. On rappelle que EG contient des interactions à deux, trois et quatres atomes.

2°) - EC est l'énergie coulombienne effective, dépendant de charges phénoménologiques attribuées à chaque atome de la molécule ; l'existence de charges phénoménologiques pouvant, entre autres, être non entières, peut être trouvée dans le concept de délocalisation de la mécanique quantique.

L'interaction d'origine coulombienne entre deux atomes i et j s'écrit sous la forme ;

$$EC = \sum_{i<j} (qiqj/4\pi\varepsilon o\varepsilon r)(Cij/rij),$$

où εr est la permittivité diélectrique du milieu dans lequel se trouve la molécule (que l'on prend égal à 1 dans le vide), εo est la permittivité diélectrique du vide,

$$rij = |\vec{ri} - \vec{rj}|$$

distance entre les atomes i et j (i<j pour ne compter qu'une fois chaque paire d'atomes en interaction).

Dans l'expression EC, les coefficients Cij traduisent le fait que EC est d'origine phénoménologique et non directement liée aux interactions microscopiques ; il existe d'ailleurs des formes d'interactions coulombiennes effectives plus complexes avec un terme de coupure correspondant à l'apparition de la quantification dans les solutions.

Ainsi, on prend Cij=0 pour des atomes trop proches dans la structure chimique (par exemple si i et j sont reliés entre eux par un angle de valence ou une liaison covalente) ; ce choix traduit le fait que l'interaction de Coulomb est déjà prise en compte dans l'énergie géométrique. Par exemple, on prend Cij=0 si les atomes i et j sont séparés par une ou deux liaisons de valence, Cij=1/2 si les atomes i et j sont séparés par trois liaisons de valence, Cij=1 si les atomes i et j sont séparés par quatre (ou plus) liaisons de valence.

Les charges qi sont elles aussi effectives et ne sont donc pas a priori un multiple entier de la charge électrique de l'électron. Par exemple, pour un atome de carbone lié à 3 hydrogènes et à un carbone, la charge effective ve est de -0,17 charge unité.

3°) - ELJ est une énergie effective de Lennard-Jones, comprenant un coeur dur répulsif à courte distance traduisant la non-pénétrabilité des atomes ; c'est une interaction attractive de type Van der Waals à grande distance. La présence du coeur dur (ou rayon de Van der Waals) interdit l'effondrement de tout ou partie de la molécule sur elle-même ; dans ELJ peuvent aussi apparaître les énergies des liaisons hydrogène.

L'énergie effective de Lennard-Jones entre deux atomes i et j est prise sous la forme :

$$ELJ = \sum_{i<j} \left(\frac{aij}{rij^{12}} - \frac{bij}{rij^{6}}\right) dij$$

où

$$rij = |\vec{ri} - \vec{rj}|$$

est la distance entre les atomes i et j.

Les coefficients de Lennard-Jones aij et bij sont déterminés de manière empirique, par exemple en imposant la position et la valeur du minimum de la fonction ($aij/r^{12}$)-($bij/r^{6}$). Les dij sont choisis de manière analogue au Cij dans l'interaction coulombienne; en particulier les dij sont nuls si les atomes i et j sont trop proches dans la structure chimique ; par exemple dij vaut 0 si i et j sont séparés par une ou deux liaisons covalentes, si non dij vaut 1.

L'énergie d'interaction de Lennard-Jones est fondamentale en particulier à courte distance car elle traduit l'impénétrabilité des atomes ; les problèmes d'encombrement stérique dans la molécule sont en effet primordiaux.

L'énergie effective de Lennard-Jones peut par ailleurs contenir un terme de la forme : ($aij/rij^{12}$)-($bij/rij^{10}$) simulant l'existence de liaisons hydrogène.

Pour conclure, la forme de l'énergie d'interaction utilisée ici est justifiée de manière phénoménologique, mais toute autre expression serait susceptible d'être utilisée de façon analogue dans le procédé de l'invention.

La forme de l'énergie d'interaction E permet de comprendre pourquoi le problème de la minimisation de cette énergie est un problème difficile. En effet, certains termes de a, b et c sont incompatibles entre eux, c'est-à-dire qu'ils ne peuvent pas être minimisés simultanément ; c'est le phénomène de frustration. Cette incompatibilité engendre une structure de l'espace des phases qui peut être très complexe et posséder de nombreux minima locaux. Cette complexité croît très vite avec le nombre N d'atomes de la molécule (exponentiellement avec N), d'où l'intérêt du procédé de l'invention.

Les différentes phases du procédé de l'invention permettant l'échantillonnage (ou étape IV) de la molécule à une température déterminée T sont les suivantes :

a) - on choisit d'abord une numérotation de 1 à N des atomes de la molécule, de manière cohérente. Il existe de nombreux choix de numérotation, la seule limite imposée à cette flexibilité est l'obligation que, si les atomes 1 à i ont été choisis, alors l'atome i+1 doit avoir une liaison covalente avec l'un au moins des i atomes précédents.

b) - on considère alors un nombre M de configurations spatiales initiales, c'est-à-dire formées d'un seul et unique atome ; pour des facilités numériques cet atome peut être choisi en bout de chaîne mais ce n'est pas nécessaire.

c) - on effectue alors la croissance des chaînes d'atomes, atome par atome ou groupe d'atomes par groupe d'atomes. Ce processus de croissance consiste à ajouter l'atome i+1 de la numérotation définie en a) à la molécule partielle formée par les i premiers atomes. Le choix de la position du nouvel atome i+1 associé à la chaîne peut être fait de multiples façons soit de manière connue, par une distribution uniforme dans les variables adéquates ou par une distribution gaussienne à l'aide de la technique de Box Muller, soit, selon l'invention, par introduction d'un champ "fantôme", etc.

d) - il est alors possible de calculer l'accroissement de l'énergie de la molécule partielle due à l'ajout de ce nouvel atome ou groupe d'atomes.

e) - la valeur de l'accroissement en énergie calculée en d) permet de répliquer (reproduire de façon identique) ou de détruire la configuration partielle de coordonnées ainsi considérée. Ce processus a pour objet de favoriser les configurations de coordonnées où l'accroissement en énergie est négatif (c'est-à-dire des configurations de basse énergie) aux dépens de celles où l'accroissement en énergie est positif (configuration d'énergie élevée). Il faut noter que toute configuration défavorable pour un atome peut se retrouver favorable quelques atomes plus loin.

Ce procédé permet d'échantillonner simultanément des minima du profil en énergie de la molécule même si ceux-ci sont séparés par des barrières en énergie très élevées. Si le nombre de configurations total est trop faible, l'échantillonnage est biaisé puisque le minimum absolu peut ne pas être représenté.

f) - un des points importants du procédé de l'invention, à part le point e) qui décrit la procédure de réplication-destruction, est la procédure qui permet de garder le nombre de configurations à un atome i donné sensiblement constant. En effet, si aucune précaution n'est prise, ce nombre peut devenir nul ou très rapidement dépasser les limites de capacité de mémorisation de toute machine de traitement et de mémorisation.

Cette procédure fait intervenir un facteur d'échelle gi à chaque ajout d'un atome ou groupe d'atomes.

g) - Une fois les phases a) à f) accomplies, l'ensemble des configurations de coordonnées obtenues de la molécule décrit est un ensemble "boltzmannien". L'étape IV, correspondant à l'échantillonnage du système, est donc achevée. On peut alors minimiser l'énergie de tout ou partie des configurations obtenues par la méthode de gradients conjugués, mémoriser puis visualiser les configurations d'énergie minimisée.

Le procédé de l'invention présente, en plus des avantages ci-dessus, l'avantage d'obtenir directement l'entropie qui est une quantité statistique, contrairement aux autres procédés connus d'échantillonnage utilisant une méthode de Monte Carlo.

La description se réfère aux figures annexées, dans lesquelles :

– la figure 1, déjà décrite, donne l'énergie de la molécule en fonction des coordonnées des atomes, et

– la figure 2 illustre un dispositif de traitement pour la mise en oeuvre du procédé conforme à l'invention.

D'autres caractéristiques et avantages de l'invention ressortiront mieux de la description qui va suivre, donnée à titre illustratif et non limitatif.

Après un exposé général de l'étape d'échantillonnage du procédé de l'invention, on développera les points originaux de ce procédé sur un exemple précis, simple mais tout à fait générique : celui de l'alanine dipeptide.

Considérons une molécule de N atomes pour lesquels on a choisi un ordre (assez largement arbitraire) de numérotation.

A cette molécule est associée une énergie d'interaction

$$E(\vec{r1},\ldots,\vec{rN})$$

qui dépend de la position $\vec{ri}$ des N atomes, avec $1 \leqq i \leqq N$.

On peut définir, pour l'atome i, l'énergie d'interaction

$$Ei(\vec{r1},\ldots,\vec{ri})$$

qui est la partie de l'énergie d'interaction E qui ne contient aucun atome (i+1,...,N). On peut la considérer comme une energie d'interaction partielle sur les i premiers atomes. On prend E1=0 par convention et on vérifie que EN=E.

L'énergie d'interaction a été précisée précédemment et le sera davantage dans l'exemple de l'alamine dipeptide.

Pour échantillonner le système statistique (étape IV), il faut réussir à produire une population MN de molécules (complète N atomes) où la configuration de coordonnées

$$(\vec{ri},\ldots,\vec{rN})$$

est présente un nombre de fois proportionnel à

$$\exp\left(-\beta E(\vec{ri},\ldots,\vec{rN})\right).$$

On commence tout d'abord par une population M1 de molécules partielles composées de l'atome 1. L'invariance par translation du système nous incite à fixer l'atome 1, par exemple à l'origine des coordonnées ; cependant, ce point est secondaire pour la démonstration.

On ajoute alors le deuxième atome. L'énergie associée au premier atome était

$$E1(\vec{r1})$$

qui est nulle et le choix de l'atome 1 fixé à l'origine est bien compatible avec ce fait.

L'énergie associée aux deux premiers atomes est

$$E2(\vec{r1},\vec{r2}) \quad ;$$

l'accroîssement en énergie est donc égal à

$$E2(\vec{r1},\vec{r2})-E1(\vec{r1})$$

que l'on peut noter

$$\Delta E2(\vec{r1},\vec{r2}).$$

On tire la position de l'atome 2 avec une loi de probabilité

$$\exp\left(-\beta V2(\vec{r1},\vec{r2})\right).$$

Le tirage de l'atome 2 n'est pas nécessairement uniforme ; en effet, les atomes 1 et 2 étant liés de manière covalente et leur énergie d'interaction d'origine covalente étant décrite par une énergie quadratique piquée à

son minimum, il est préférable de choisir $\vec{r2}$, de sorte que

$$\vec{r2}-\vec{r1}$$

soit distribuée de manière gaussienne autour du minimum.

On considère alors la quantité proportionnelle au poids de Boltzmann, notée

$$W2(\vec{r2}|\vec{r1})=\exp(-\beta(\Delta E2(\vec{r1},\vec{r2})-V2(\vec{r1},\vec{r2})))$$

et on réplique la configuration définie par les positions

$$(\vec{r1},\vec{r2})$$

avec le poids

$$W2(\vec{r2}|\vec{r1}).$$

V2 est une fonction qui permet de tirer $\vec{r2}$ avec une distribution de probabilité correctement biaisée.

De manière explicite, on calcule la partie entière de

$$W2(\vec{r2}|\vec{r1})=iW$$

et rW=W2-iW le reste.

Si iW est plus grand que 1, on réplique la configuration

$$(\vec{r1},\vec{r2})$$

iW fois (c'est-à-dire que dans l'ensemble des configurations à 2 atomes, elle apparait iW fois), ensuite on tire un nombre r au hasard entre 0 et 1 de manière uniforme, par exemple à l'aide d'un générateur de nombres aléatoires présents dans l'unité de traitement et de mémorisation.

Si r<rW, alors on ajoute une configuration de plus (c'est-à-dire que l'on en a iW+1 dans l'ensemble statistique), si rW<r alors on ne l'ajoute pas.

Si W2 est plus petit que 1, alors on tire un nombre r au hasard entre 0 et 1 de manière uniforme. Si r<rW alors on met une configuration

$$(\vec{r1},\vec{r2})$$

dans l'ensemble statistique, si rW<r, alors la configuration

$$(\vec{r1},\vec{r2})$$

disparaît de l'ensemble : elle est détruite.

Si on calcule la distribution à laquelle obéissent alors les molécules à deux atomes ainsi construites, on constate qu'elle obéit à la loi :

$$\exp(-\beta\Delta E2(\vec{r1},\vec{r2}))=\exp(-\beta E2(\vec{r1},\vec{r2})).$$

Le nombre de configurations de molécules formées de deux atomes dans l'ensemble statistique déterminé par les coordonnées

$$(\vec{r1},\vec{r2})$$

est égal à

$$M2(\vec{r1},\vec{r2})=M1\exp(-\beta E2(\vec{r1},\vec{r2})).$$

La procédure est itérative. On suppose qu'au stade i, la population totale de l'ensemble de configurations est M1 et que la population de configurations de coordonnées

$$(\vec{r1},...,\vec{ri})$$

est :

$$Mi(\vec{r1},...,\vec{ri}) = M1\exp(-\beta Ei(\vec{r1},...,\vec{ri})).$$

On veut alors ajouter l'atome i+1 dont on tire la position

$$\vec{ri+1}$$

avec une loi de probabilité judicieuse

$$\exp(-\beta Vi+1(\vec{r1},...,\vec{ri+1})).$$

On considère alors la quantité proportionnelle au poids de Boltzmann, notée :

$$Wi+1(\vec{ri+1}|\vec{r1},...,\vec{ri})=$$

$$\exp(-\beta(\Delta Ei+1(\vec{r1},...\vec{ri+1})-Vi+1(\vec{r1},...,\vec{ri+1}))),$$

$$\text{où } \Delta Ei+1(\vec{r1},...,\vec{ri+1})=Ei+1(\vec{r1},...,\vec{ri+1})-Ei(\vec{r1},...,\vec{ri}).$$

On réplique alors la configuration de coordonnées

$$(\vec{r1},...,\vec{ri+1})$$

avec le poids Wi+1 comme cela a été expliqué précédemment. Le nombre de configurations définies par les coordonnées

$$(\vec{r1},...\vec{ri+1})$$

est donné par :

$$Mi+1(\vec{r1},...,\vec{ri+1})=Mi(\vec{r1},...,\vec{ri})\exp(-\beta\Delta Ei+1(\vec{r1},...,\vec{r1+1})),$$

soit par récurrence :

$$Mi+1(\vec{r1},...,\vec{ri+1})=M1\exp(-\beta Ei+1(\vec{r1},...,\vec{ri+1})).$$

Dans le cas où i+1=N, on obtient :

$$MN(\vec{ri},...,\vec{rN})=M1\exp(-\beta E(\vec{r1},...,\vec{rN})).$$

Cette identité exprime que l'ensemble des configurations finales de coordonnées est distribué de manière "boltzmannienne" puisque chaque configuration de coordonnées apparaît proportionnellement à son poids de Boltzmann.

Un problème évident de la procédure précédente est qu'elle conduit à une croissance exponentielle ou à une décroissance de la population totale Mi à l'atome i.

Il est facile de vérifier que l'introduction d'un facteur d'échelle gi commun à toutes les configurations, au niveau de l'atome i :

$$Wi(\vec{ri}|\vec{r1},...,\vec{ri}-1)=gi\exp\left(-\beta\left(\Delta Ei(\vec{r1},...,\vec{ri})-Vi(\vec{r1},...\vec{ri})\right)\right)$$

ne modifie pas le raisonnement fait précédemment et conduit à la relation :

$$Mi+1(\vec{r1},...,\vec{ri}+1)=M1g1,...,gi+1\exp\left(-\beta Ei+1(\vec{r1},...,\vec{ri}+1)\right).$$

Un choix judicieux des facteurs d'échelle permet de contrôler les fluctuations de la population de l'ensemble des configurations.

On peut remarquer que la définition des énergies

$$Ei(\vec{r1},...,\vec{ri})$$

est assez arbitraire. Cet arbitraire peut permettre de biaiser les distributions des configurations durant la croissance, sachant qu'à la fin du processus de croissance, il y a la contrainte :

$$EN(\vec{r1},...,\vec{rN})=E(\vec{r1},...,\vec{rN}).$$

De plus, on peut noter que l'entropie ou l'énergie se calcule très facilement à partir de la formule

$$MN(\vec{r1},...,\vec{rN})=M1g1,...,gN\exp(-\beta E(\vec{r1},...,\vec{rN})).$$

En effet, si MN est la population finale des configurations, on a :

$$Z=\sum\exp(-\beta E(\vec{r1},...,\vec{rN}))=M1(\vec{g1},...,\vec{gN})^{-1}.MN.$$

Le procédé de l'invention peut être mis en oeuvre avec le dispositif de traitement représenté sur la figure 2.

Ce dispositif comprend un thermostat T pour fixer la température à laquelle on veut obtenir les configurations spatiales les plus stables de la molécule que l'on étudie, connecté à une unité centrale UC de traitement.

Cette unité centrale UC est connectée à un clavier C pour l'introduction des données (température, structure chimique, énergies et interations choisies et mise en jeu entre les différents atomes de la molécule), à une mémoire $M_1$ pour la mémorisation de ces données, à une mémoire $M_2$ et à un circuit S du type ordinateur pour générer les tracés de Ramachandran.

L'unité centrale UC, les mémoires $M_1$ et $M_2$ et le circuit S peuvent être regroupés dans un même ordinateur par exemple ceux du type Alliant Convex(R), Cray(R), Silicon Graphics(R), Sun(R), etc, ou bien être réalisés avec des circuits distincts reliés entre eux par des bus bidirectionnels.

L'unité centrale UC permet la formation d'un échantillon statistique de l'ensemble de Boltzmann des configurations des coordonnées (ou agencement dans l'espace) des atomes de la molécule à étudier selon le processus de croissance, réplication, supression (étape IV). Elle assure en outre l'ajustement de population pour qu'elle reste sensiblement constante au cours de son élaboration.

L'échantillon statistique obtenu est ensuite visualisé sur le dispositif de visualisation V. On trace alors des tracés de Ramachandran sur le dispositif de visualisation générés à partir des circuits, et on sélectionne grâce aux circuits une configuration de coordonnées dans chacun de ces tracés.

Le circuit S minimise alors l'énergie des configurations ainsi sélectionnées selon la technique de gradients conjugués décrite par exemple dans le document de Z. Li et H.A. Sheraza cité plus haut.

Les configurations de coordonnées d'énergie les plus basses obtenues sont stockées dans la mémoire $M_2$. Elles peuvent aussi être visualisées sur le dispositif de visualisation.

En faisant alors correspondre à chaque type d'atome et de liaisons interatomiques une taille et une couleur, on peut alors visualiser sur le dispositif V les agencenents spatiaux des atomes de la molécule les plus probables.

En changeant la température du thermostat, on peut alors voir les atomes de la molécules se mouvoir sur le dispositif V (et observer ainsi les transitions de repliement des protéines).

EXEMPLE : l'alanine dipeptide.

On illustre ci-après l'étape IV du procédé de l'invention sur la molécule d'alanine dipeptide.

Cette molécule est représentée par la formule (II) suivante :

(II)

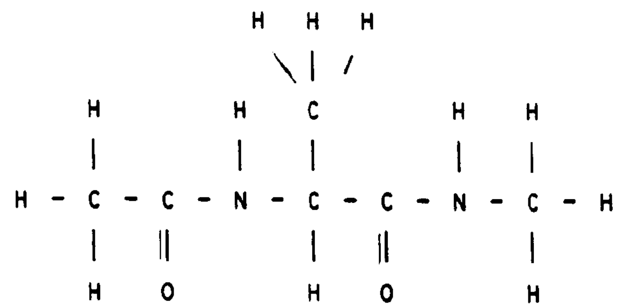

α) - La numérotation des atomes.

Le choix de la numérotation est arbitraire, aux réserves près faites précédemment. Cependant, pour des raisons liées à l'encombrement stérique, il semble préférable de faire croître la protéine de telle sorte que les différentes parties des résidus (chaines latérales) croissent de manière synchrone.

Une numérotation possible est donnée ci-après pour la croissance des chaînes d'atomes.

(II)

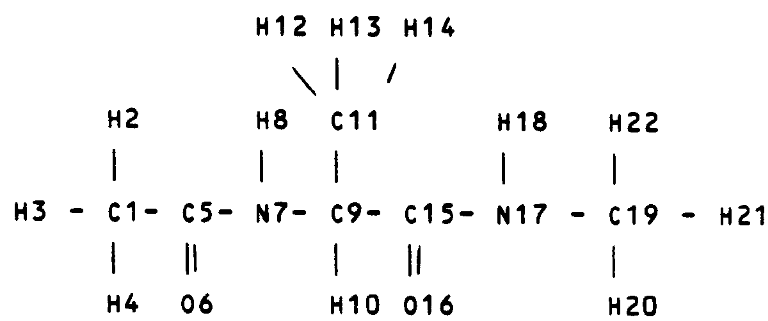

β) - Les paramètres à entrer dans la mémoire $M_1$.

Il faut fournir les différents types chimiques des atomes : en effet, un carbone lié à un oxygène n'a pas les mêmes propriétés qu'un carbone tétraédrique ; de même un hydrogène lié à un azote n'a pas les mêmes propriétés qu'un hydrogène lié à un carbone.

Il faut ensuite fournir tous les paramètres physiques mentionnés précédemment qui permettent de calculer l'énergie d'interaction des atomes de la protéine. Ces paramètres peuvent être modifiés ; il en existe d'ailleurs plusieurs jeux. Ainsi, le choix des angles diédres n'est pas unique pour définir l'énergie d'interaction phénoménologique.

γ) - Le processus de croissance avec l'unité centrale UC.

- L'atome 1 : C

On considére M1 copies du C portant le numéro 1 ; pour des raisons d'invariance, par translation, on le fixe à l'origine

$$(\vec{r_1} = 0)$$

d'un repére orthonormé ox,y,z et son énergie est prise comme référence d'où

$$E1(\vec{r1})=0.$$

Dans ce repère, xi, yi et zi représentent les coordonnées spatiales de l'atome i de la molécule.

- L'atome 2 : H

Sa position est $\vec{r2}$ et d'aprés les définitions ci-dessus :

$$E2(\vec{r1},\vec{r2}) = K12(r12-r^{o}12)^{2}, \text{ où } r12=|\vec{r2}-\vec{r1}|.$$

On prend alors

$$V2(\vec{r1},\vec{r2}) = K12(r12-r^{o}12)^{2} ;$$

en effet, la méthode de Box Muller permet de tirer les configurations

$$(\vec{r1}-\vec{r2})$$

avec la distribution de probabilité gaussienne

$$\exp(-\beta V2(\vec{r1},\vec{r2})).$$

La quantité

$$V2(\vec{r2}|\vec{r1}) = g2\exp(-\beta(\Delta E2(\vec{r1},\vec{r2})-V2(\vec{r1},\vec{r2})))$$

est indépendante de

$$(\vec{r1},\vec{r2}) ;$$

on la prend égale à 1 pour conserver la population totale constante, soit M1.

Seule la longueur

$$|\vec{r1}-\vec{r2}|$$

est tirée, pour des raisons liées à l'invariance par rotation de la protéine dans l'espace. On peut prendre par exemple x2=0, y2=0, z2=r12.

- L'atome 3 : H

Sa position est $\vec{r3}$ et d'aprés les définitions ci-dessus :

$$E3(\vec{r1},\vec{r2},\vec{r3})=K12(r12-r^{o}12)^{2} + K13(r13-r^{o}13)^{2}+K213(\theta213-\theta^{o}213)^{2},$$

où $\theta\widehat{213}$ est l'angle de valence $\widehat{213}$ et

$$r13=|\vec{r1}-\vec{r3}|,$$

et

$$\Delta E3(\vec{r1},\vec{r2},\vec{r3})=E3(\vec{r1},\vec{r2},\vec{r3})-E2(\vec{r1},\vec{r2})=K13(r13-r^{\circ}13)^2+K\widehat{213}(\theta\widehat{213}-\theta^{\circ}\widehat{213}).$$

C'est une expression quadratique ; on peut donc prendre, comme pour l'atome 2 :

$$V3(\vec{r1},\vec{r2},\vec{r3})=\Delta E3(\vec{r1},\vec{r2},\vec{r3}).$$

Ceci entraîne que

$$W3(\vec{r3},|\vec{r1},\vec{r2})$$

est constant; on le prend égal à 1 pour conserver la population totale, soit M1.

Pour des raisons d'invariance par rotation, on peut prendre $\vec{r3}$ dans le plan yz ; c'est-à-dire x3=0.

- L'atome 4 : H

Sa position est $\vec{r4}$ et d'après les définitions précédentes, on a :

$$E4(\vec{r1},\vec{r2},\vec{r3},\vec{r4})=K12(r12-r^{\circ}12)^2+K13(r13-r^{\circ}13)^2+K\widehat{213}(\theta\widehat{213}-\theta^{\circ}\widehat{213})^2+K14(r14,r^{\circ}14)^2+K\widehat{214}(\theta\widehat{214}-\theta\widehat{214})^2+K\widehat{314}(\theta\widehat{314}-\theta^{\circ}\widehat{314})^2,$$

où

$$r14=|\vec{r1}-\vec{r4}|$$

et $\theta\widehat{214}$ et $\theta\widehat{314}$ sont les angles de valence respectivement $\widehat{314}$ et $\widehat{214}$ et

$$\Delta E4(\vec{r1},\vec{r2},\vec{r3},\vec{r4})=K14(r14-r^{\circ}14)^2+K\widehat{214}(\theta\widehat{214}-\theta^{\circ}\widehat{214})^2+K\widehat{314}(\theta\widehat{314}-\theta^{\circ}\widehat{314})^2;$$

c'est encore une expression quadratique.

Il est aisé de tirer $\theta\widehat{214}$ et $\theta\widehat{314}$ de manière gaussienne par la méthode de Box Muller pour en déduire $\vec{r4}$. Ainsi on prend encore :

$$V4(\vec{r1},\vec{r2},\vec{r3},\vec{r4})=\Delta E4(\vec{r1},\vec{r2},\vec{r3},\vec{r4}).$$

On remarque que les valeurs de r14, $\theta\widehat{214}$ et $\theta\widehat{314}$ distribuées de manière gaussienne sélectionnent deux valeurs possibles de $\vec{r4}$.

En effet, 2, 3, 4 sont les sommets d'un tétraèdre de centre 1. On choisit une des deux solutions à chaque configuration pour des raisons de symétrie. Ce choix n'a pas d'importance.

- L'atome 5 : C

Sa position est $\vec{r5}$ et d'après les définitions ci-dessus :

$$\begin{aligned} E5(\vec{r1},\vec{r2},\vec{r3},\vec{r4},\vec{r5}) &= K12(r12-r^{\circ}12)^2+K13(r13-r^{\circ}13)^2 \\ &+K\widehat{213}(\theta\widehat{213}-\theta^{\circ}\widehat{213})^2+K14(r14,r^{\circ}14)^2 \\ &+K\widehat{214}(\theta\widehat{214}-\theta\widehat{214})^2 \\ &+K\widehat{314}(\theta\widehat{314}-\theta^{\circ}\widehat{314})^2+K15(r15-r^{\circ}15)^2 \\ &+K\widehat{215}(\theta\widehat{215}-\theta^{\circ}\widehat{215})^2 \\ &+K\widehat{315}(\theta\widehat{315}-\theta^{\circ}\widehat{315})^2+K\widehat{415}(\theta\widehat{415}-\theta^{\circ}\widehat{415})^2 \end{aligned}$$

avec

$$r15=|\bar{r1}-\bar{r5}|$$

et

$$\theta\widehat{315},\ \theta\widehat{215},\ \theta\widehat{415}$$

les angles de valence respectivement

$$\widehat{315},\ \widehat{215}$$

et $\widehat{415}$ et

$$\begin{aligned} \Delta E5(\vec{r1},\vec{r2},\vec{r3},\vec{r4},\vec{r5}) &= E5(\vec{r1},\vec{r2},\vec{r3},\vec{r4},\vec{r5})-E4(\vec{r1},\vec{r2},\vec{r3},\vec{r4}) \\ &=K15(r15-r^{\circ}15)^2+K\widehat{215}(\theta\widehat{215}-\theta^{\circ}\widehat{215})^2 \\ &+K\widehat{315}(\theta\widehat{315}-\theta^{\circ}\widehat{315})^2+K\widehat{415}(\theta\widehat{415}-\theta^{\circ}\widehat{415})^2. \end{aligned}$$

L'expression ΔE5 est toujours quadratique mais malheureusement les angles

$$\theta\widehat{215},\quad \theta\widehat{315}$$

et $\theta\widehat{415}$ ne sont pas indépendants ; en effet la donnée de θ215 et de $\theta\widehat{315}$ détermine complètement la

valeur de $\theta\widehat{415}$.

On choisit alors

$$V5(\vec{r1},\vec{r2},\vec{r3},\vec{r4},\vec{r5})$$

qui ne contient que $\theta\widehat{215}$ et $\theta\widehat{315}$ ; ainsi l'expression est quadratique et la distribution exp(-βV5) peut être calculée à l'aide de la méthode de Box Muller.

D'où :

$$V5(\vec{r1},\vec{r2},\vec{r3},\vec{r4},\vec{r5})$$
$$=K15(r15-r^{o}15)^{2}+K\widehat{215}(\theta\widehat{215}-\theta^{o}\widehat{215})^{2}+K\widehat{315}(\theta\widehat{315}-\theta^{o}\widehat{315})^{2}$$

avec lequel on tire r15,

$$\theta\widehat{215},\ \theta\widehat{315}$$

qui déterminent complètement la position de l'atome 5. Dans le cas présent il y a 2 solutions possibles, une solution qui compléte le tétraèdre et l'autre qui percute l'atome 4, c'est-à-dire que les atomes 4 et 5 occupent la même position.

Aussi,

$$W5(\vec{r5}|\vec{r1},\vec{r2},\vec{r3},\vec{r4})=g5\exp(-\beta K\widehat{415}(\theta\widehat{415}-\theta^{o}\widehat{415})^{2})$$

est le facteur de réplication ; les valeurs numériques usuelles permettent de montrer que la solution de l'atome 5 qui percute l'atome 4 est exclue systématiquement ; en effet, la valeur de $\theta\widehat{415}$ vaut alors zéro et la quantité

$$\beta K\widehat{415}\ \theta^{o}\widehat{415}^{2},$$

est grande devant l'unité.

- L'atome 6 : 0

Sa position est $\vec{r6}$, et d'après les définitions précédentes :

$$E6(\vec{r1},\vec{r2},\vec{r3},\vec{r4},\vec{r5},\vec{r6})=E5(\vec{r1},\vec{r2},\vec{r3},\vec{r4},\vec{r5})+K56(r56-r^{o}56)^{2}$$
$$+K\widehat{156}(\theta\widehat{156}-\theta^{o}\widehat{156})^{2}+E6^{C}+E6^{LJ},$$

où $E6^{C}$ et $E6^{LJ}$ représentent respectivement les énergies d'interaction de Coulomb et de Lennard-Jones.

Celles-ci ne posent pas de question de principe et ne sont jamais incorporées dans la fonction Vi donc ici dans la fonction V6.

r56 et $\theta\widehat{156}$ sont définis comme précédemment.

On pourrait tirer r56 et $\theta\widehat{156}$ de manière gaussienne (c'est-à-dire faire : $V6=K56(r56-r^{o}56)^{2}$

$$+K\widehat{156}(\theta\widehat{156}-\theta^{o}\widehat{156})^{2})$$

et répliquer à l'aide des interactions de Coulomb et de Lennard-Jones (c'est-à-dire que W6=g6exp(-$\beta$($E6^C$+$E6^{LJ}$)).

En fait, ce choix donne un atome d'oxygène 6 dégénéré sur un cône.

Or, on sait que les atomes 1, 5, 6, 7 sont presque coplanaires et que, d'autre part, les atomes 2, 1, 5, 7 sont liés entre eux par un angle dièdre, toutes conditions qui font que le choix de la position de l'atome 6 précédent sera certainement incompatible avec la position de l'atome 7 à venir. En effet, la position de cet atome 7 est définie indépendamment de l'atome 6, par exemple, par l'angle de valence $\widehat{157}$ et par l'angle dièdre $\widehat{2157}$.

On considére alors, pour remédier à ce problème, le choix d'un "pseudo" atome 7 à l'aide d'un angle de valence $\widehat{157}$ et d'un angle dièdre (par exemple $\widehat{2157}$). Ce choix peut être fait à l'aide de la distribution de probabilité déduite de la valeur de l'énergie d'interactions (il faut alors utiliser une méthode de réjection de Von Neumann car la probabilité n'est plus gaussienne à cause du terme dièdre) ou bien toute autre méthode qui fixera le "pseudo" atome 7 à proximité de sa position de plus forte probabilité. On peut par exemple prendre

$$\theta\widehat{157}=\theta^\circ\widehat{157}$$

et

$$\theta\widehat{2157}=\theta^\circ\widehat{2157}.$$

Une fois ce "pseudo" atome 7 choisi, on peut écrire :

$$V6(\vec{r1},...,\vec{r6}) = K56(r56-r^\circ 56)^2 + K\widehat{156}(\theta\widehat{156}-\theta^\circ\widehat{156})^2 + K\widehat{756}(\theta\widehat{756}-\theta^\circ\widehat{756})^2.$$

D'où :

$$W6(r6|\vec{r1},...,\vec{r5})=g6exp(-\beta(E6^C+E6^{LJ}-K\widehat{756}(\theta\widehat{756}-\theta^\circ\widehat{756})^2)).$$

Le terme de tirage exp(-$\beta$V6) va imposer à l'atome 6 de ne pas être dégénéré sur le cône mentionné précèdemment et le terme W6 fait disparaître la référence à l'atome 7.

Une telle procédure est indispensable pour l'efficacité du procédé de l'invention. Elle consiste en particulier à guider l'atome vers la position qu'il devra statistiquement occuper même si, au stade de la croissance où il est, il pourrait occuper d'autres positions. Cette procédure est appelée le champ "fantôme".

Il faut remarquer que l'atome 7 disparait complètement à la fin de l'étape de construction de l'atome 6.

- L'atome 7 : N

Sa position est $\vec{r7}$ et par référence à ce qui précède on prend :

$$V7(\vec{r1},..., \vec{r7})=K57(r57-r^\circ 57)^2+ K157(\theta 157-\theta^\circ 157)^2 + K657(\theta\widehat{657}-\theta^\circ\widehat{657})^2.$$

D'où :

$$W7(\vec{r7}|_{G'}\vec{r1},\ldots,\vec{r6})=g7\exp(-\beta(E7^{C}+E7^{LJ}+E7^{G'})),$$

où $E7^{G'}$ comprend l'énergie d'interaction des dièdres impropres $(\widehat{5176})$ et des dièdres propres

$$(\widehat{2157});\ (\widehat{3157})$$

et $(\widehat{4157})$. On remarque qu'avec V7, la position de l'atome est déterminée ; en effet, on a deux angles et une longueur.

- L'atome 8 - H

On a besoin ici d'un champ "fantôme" induit par l'atome 9.

- L'atome 9 est un carbone chiral. Or, les protéines naturelles sont constituées à partir d'acides aminés gauches. Ceci implique une position relative des atomes 7, 9, 10, 11, 15 unique.

- L'atome 10 : H

On a encore besoin d'un champ "fantôme" induit par l'atome 15. Il faut choisir la solution telle que le tétraèdre 7, 9, 10, 11, 15 soit gauche. Autrement dit, il ne faut pas permuter les atomes 10 et 11.

La fin de la chaîne s'effectue sans difficulté supplémentaire.

Par ailleurs, la contrainte de chiralité des carbones asymétriques est inessentielle à la mise en oeuvre de l'invention.

δ) - Le cas des interactions à distance.

Les interactions à distance comprennent les interactions coulombiennes et celles de Lennard-Jones. La flexibilité du procédé de l'invention (c'est-à-dire le choix des énergies Ei) permet de les répartir de façon variée. Le choix le plus judicieux semble être d'associer l'interaction de Lennard-Jones à chaque étape du processus de croissance. En ce qui concerne les interactions de Coulomb, la valeur très forte des énergies obtenues pour les groupes non neutres d'atomes incite à les associer aux seuls atomes i tels que le sous-groupe d'atomes formé des atomes 1 à i soit neutre.

ε) - Le contrôle de la population des chaînes à l'aide de l'unité centrale.

On suppose qu'à l'atome i la population totale est Mi ; la procédure correspondante est donnée ci-aprés.

On fait tout d'abord un tirage de la position de l'atome i+1 avec le poids exp(-βVi+1), pour toutes les configurations de coordonnées. On conserve alors la séquence de nombres aléatoires utilisée pour effectuer ce tirage de l'atome i+1. On calcule alors la valeur minimale de la quantité (ΔE i+1-Vi+1) pour tous les tirages de la position

$$\vec{r}_{i+1}$$

et on la note $Ei+1^{min}$.

Sachant que le facteur de réplication est défini à une constante multiplicative près, identique pour toutes les configurations, on effectue le même tirage de la position de l'atome i+1 avec le poids exp(-βVi+1).

Pour une réplication avec la probabilité $Wi+1 = \exp(-\beta(\Delta Ei+1-Vi+1-Ei+1^{min}))$, du fait que l'exposant est positif, on est certain que la population de chaînes ne va pas exploser.

Avec ce poids de réplication, on peut calculer la nouvelle population notée $Mi+1^{N}$ ; le choix définitif du poids de réplication est alors, pour garder une population sensiblement égale à Mi,

$$W_{i+1} = \frac{M_i}{M_{i+1}^{N}} \exp(-\beta(\Delta E_{i+1} - V_{i+1} - E_{i+1}^{min})).$$

La procédure est toujours effectuée avec la même série de nombres aléatoires ; on a donc affaire à une méthode de type Monte Carlo.

Le procédé de l'invention présente un grand nombre d'avantages. En particulier, ce procédé présente une grande souplesse d'utilisation.

L'un des principaux avantages de ce procédé est qu'il échantillonne simultanément tout l'espace des phases, évitant de se laisser piéger dans des minima locaux. Cette assertion est toutefois liée à la valeur du paramétre N/M (longueur de la molécule biologique sur le nombre de configurations).

De plus, le procédé de l'invention est très performant en temps de calcul. Ceux-ci croissent en $N^2$ en fonction de la taille de la molécule.

Par ailleurs, les temps de calcul peuvent être considérablement améliorés en jouant sur la flexibilité du procédé et en particulier sur :

- le choix de la numérotation,
- le choix des Ei et des Vi (en particulier pour l'énergie de Coulomb),
- le choix de la procédure de croissance (on peut imaginer d'ajouter plusieurs atomes à la fois, voire des résidus entiers),
- le choix de la loi de tirage de l'atome i,
- le choix des variables dynamiques (on peut, par exemple, fixer la valeur de certaines quantités à leurs valeurs nominales ; par exemple

$$\theta\widehat{123} = \theta^{\circ}\widehat{123},$$

ce qui, par exemple, rigidifie la structure).

On note par ailleurs, que le procédé de l'invention ne part pas, a priori, d'une configuration déterminée de la molécule mais qu'il les construit de toute pièce contrairement aux procédés traditionnels où la configuration initiale est gardée en mémoire par le procédé.

Enfin, on rappelle qu'au stade final du procédé, la distribution des populations de chaînes d'atomes dans l'ensemble statistique est "boltzmannienne" donnant ainsi facilement accès au calcul de quantité d'essence statistique (valeur moyenne d'observable comme l'énergie ou l'entropie par exemple).

Le dispositif et le procédé de l'invention permettent d'étudier la transition de repliement des protéines complexes. En effet, on sait depuis quelques temps que les protéines complexes sont repliées sur elles-mémes et que ce repliement est fonction de la température à laquelle sont portées ces protéines. En outre, selon ce repliement, les centres actifs de la molécule sont différents.

Ainsi, à partir de la visualisation des conformations spatiales les plus probables et donc les plus stables des protéines à une température déterminée, il est possible de déterminer leur centre actif et de préparer ainsi de nouveaux médicaments. En particulier, grâce à l'invention, il est possible d'étudier l'action de certains médicaments sur la gangue entourant le virus du Sida, ce qui contribuera à la fabrication d'un médicament efficace contre ce virus.

L'invention présente donc un grand intérèt industriel tant dans le domaine biologique que dans le domaine pharmacologique.

## Revendications

1. Dispositif de traitement pour obtenir les conformations spatiales tridimensionnelles d'énergie les plus basses d'une molécule à l'équilibre thermodynamique, à partir de l'enchaînement de ses atomes, comprenant :
   - un thermostat (T) pour fixer la température à laquelle on veut obtenir lesdites conformations,
   - des moyens de mémorisation ($M_1$) des énergies d'interaction mettant en jeu les différents atomes de la molécule,
   - des moyens (UC) pour faire croître une population de chaînes d'atomes, atome par atome ou groupe d'atomes par groupe d'atomes, calculer l'énergie partielle des chaînes à chaque ajout d'un atome ou d'un groupe d'atomes, calculer le poids de Boltzmann relatif de chacune des configurations résultant de la croissance et répliquer ou supprimer chacune des configurations résultant de la croissance proportionnellement au poids de Boltzmann calculé, afin de former un échantillon statistique de l'ensemble de Boltzmann des configurations de coordonnées des atomes de la molécule,
– des moyens pour minimiser (S, UC, V) l'énergie de configurations dudit échantillon statistique, judicieusement choisies,
– des moyens de mémorisation ($M_2$) des configurations de coordonnées d'énergie minimisée, constituant les conformations tridimensionelles d'énergie les plus basses de ladite molécule,
– des moyens de visualisation (V) desdites conformations d'énergie les plus basses.

**2.** Dispositif selon la revendication 1, caractérisé en ce que les moyens pour minimiser comprennent des moyens (S) pour réaliser des tracés de Ramachandran des configurations dudit ensemble, pour choisir une configuration dans chacun desdits tracés et pour minimiser l'énergie des configurations ainsi choisies.

**3.** Dispositif selon la revendication 1 ou 2, caractérisé en ce que des moyens (UC) sont prévus pour ajuster la population des chaînes d'atomes à chaque ajout d'atomes ou de groupes d'atomes de sorte qu'elle reste sensiblement constante.

**4.** Dispositif selon l'une quelconque des revendications 1 à 3, caractérisé en ce qu'il comprend des moyens (UC) pour déterminer le nombre Mi+1 de configurations pour un atome i+1, avec 1≦i≦N où N est le nombre d'atomes de la molécule de façon que ce nombre satisfasse à la relation :

$$Mi+1(\vec{r1},...,\vec{ri}+1)=M1exp(-\beta Ei+1(\vec{r1},...,\vec{ri}+1))$$

où M1 est la population initiale de l'atome 1, β vaut 1/KT avec K la constante de Boltzmann et T la température à laquelle on veut obtenir les conformations, Ei+1 est l'énergie partielle des chaînes de i+1 atomes et

$$\vec{r1},...,\vec{ri}+1$$

représentent les vecteurs positions respectivement des atomes 1,...i+1 desdites chaînes.

**5.** Dispositif selon l'une quelconque des revendications 1 à 4, caractérisé en ce que des moyens sont prévus pour guider l'atome ou le groupe d'atomes que l'on ajoute pour réaliser les chaînes vers une position vers laquelle il se trouve situé du fait de contraintes géométriques.

**6.** Procédé d'obtention des conformations spatiales tridimensionnelles d'énergie les plus basses d'une molécule à l'équilibre thermodynamique, à partir de la structure chimique de cette molécule consistant à :
I) - entrer la structure chimique dans des moyens de mémorisation ($M_1$),
II) - déterminer les énergies d'interaction mettant en jeu les différents atomes de la molécule et les entrer dans lesdits moyens de mémorisation ($M_1$),
III) - fixer à l'aide d'un thermostat (T) la température à laquelle on veut obtenir lesdites conformations,
IV) - faire croître dans lesdits moyens de traitement (UC) une population de chaînes d'atomes, atome par atome ou groupe d'atomes par groupe d'atomes, en calculant l'énergie partielle des chaînes à chaque ajout d'un atome ou d'un groupe d'atomes, en calculant le poids de Boltzmann relatif de chacune des configurations résultant de la croissance et en répliquant ou en supprimant chacune des configurations résultant de la croissance proportionnellement au poids de Boltzmann calculé, afin de réaliser un échantillon statistique de l'ensemble de Boltzmann des configurations de coordonnées des atomes de la molécule,
V) - minimiser l'énergie de configurations dudit échantillon, judicieusement choisies dans les moyens de traitement (UC),
VI) - mémoriser dans des moyens de mémorisation ($M_2$) les configurations de coordonnées obtenues en V), constituant les conformations tridimentionnelles d'énergies les plus basses de ladite molécule,
VII) - visualiser les conformations d'énergies les plus basses, obtenues en VI, sur les moyens de visualisation (V).

**7.** Procédé selon la revendication 6, caractérisé en ce que l'étape V) consiste à réaliser des tracés de Ramachandran des configurations dudit échantillon sur des moyens de visualisation (V) ; choisir sur les moyens de visualisation (V) une configuration dans chacun desdits tracés.

**8.** Procédé selon la revendication 6 ou 7, caractérisé en ce que l'on ajuste dans les moyens de traitement

(UC) la population des chaînes d'atomes à chaque ajout d'atomes ou de groupes d'atomes de sorte qu'elle reste sensiblement constante.

**9.** Procédé selon l'une quelconque des revendications 6 à 8, caractérisé en ce que le nombre Mi+1 de configurations pour un atome i+1, avec $1 \leqq i \leqq N$ où N est le nombre d'atomes de la molécule, est déterminé de façon à satisfaire à la relation :

$$Mi+1(\vec{r1},\ldots,\vec{ri}+1)=M1\exp(-\beta Ei+1(\vec{r1},\ldots,\vec{ri}+1))$$

où M1 est la population initiale de l'atome 1, β vaut 1/KT avec K la constante de Boltzmann et T la température à laquelle on veut obtenir les conformations, Ei+1 est l'énergie partielle des chaînes de i+1 atomes et

$$\vec{r1},\ldots,\vec{ri}+1$$

représentent les vecteurs positions respectivement des atomes 1,...i+1 desdites chaînes.

**10.** Procédé selon l'une quelconque des revendications 6 à 9, caractérisé en ce que l'on guide l'atome ou le groupe d'atomes que l'on ajoute pour réaliser les chaînes vers une position vers laquelle il se trouve situé du fait de contraintes géométriques.

**11.** Procédé selon l'une quelconque des revendications 6 à 10, caractérisé en ce que la molécule est une protéine, un acide nucléique (ADN, ARN, ...), un polymére.

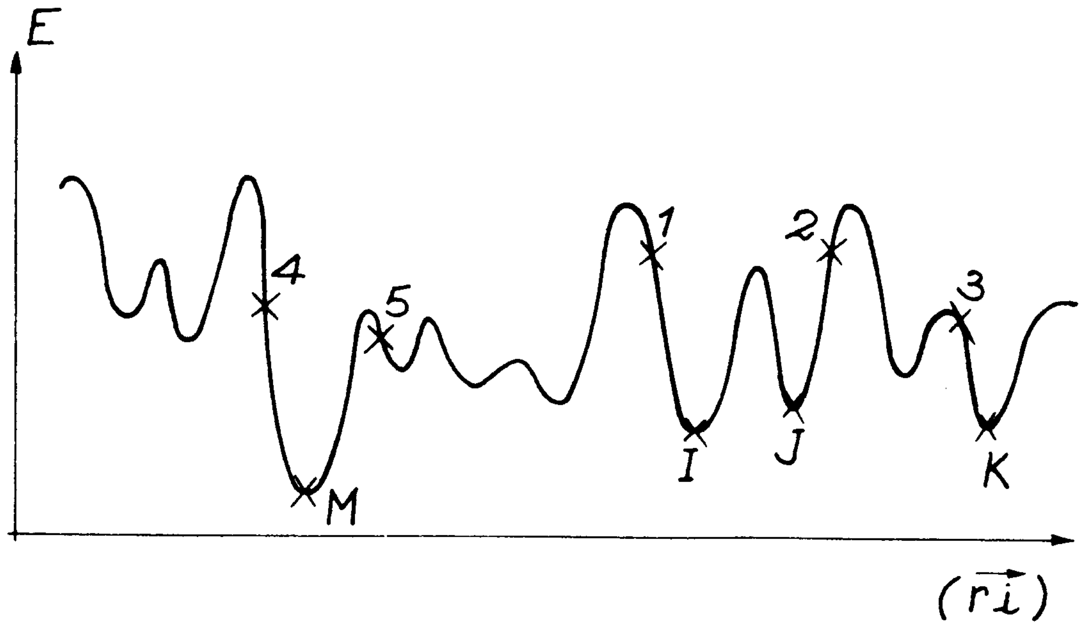


FIG. 1

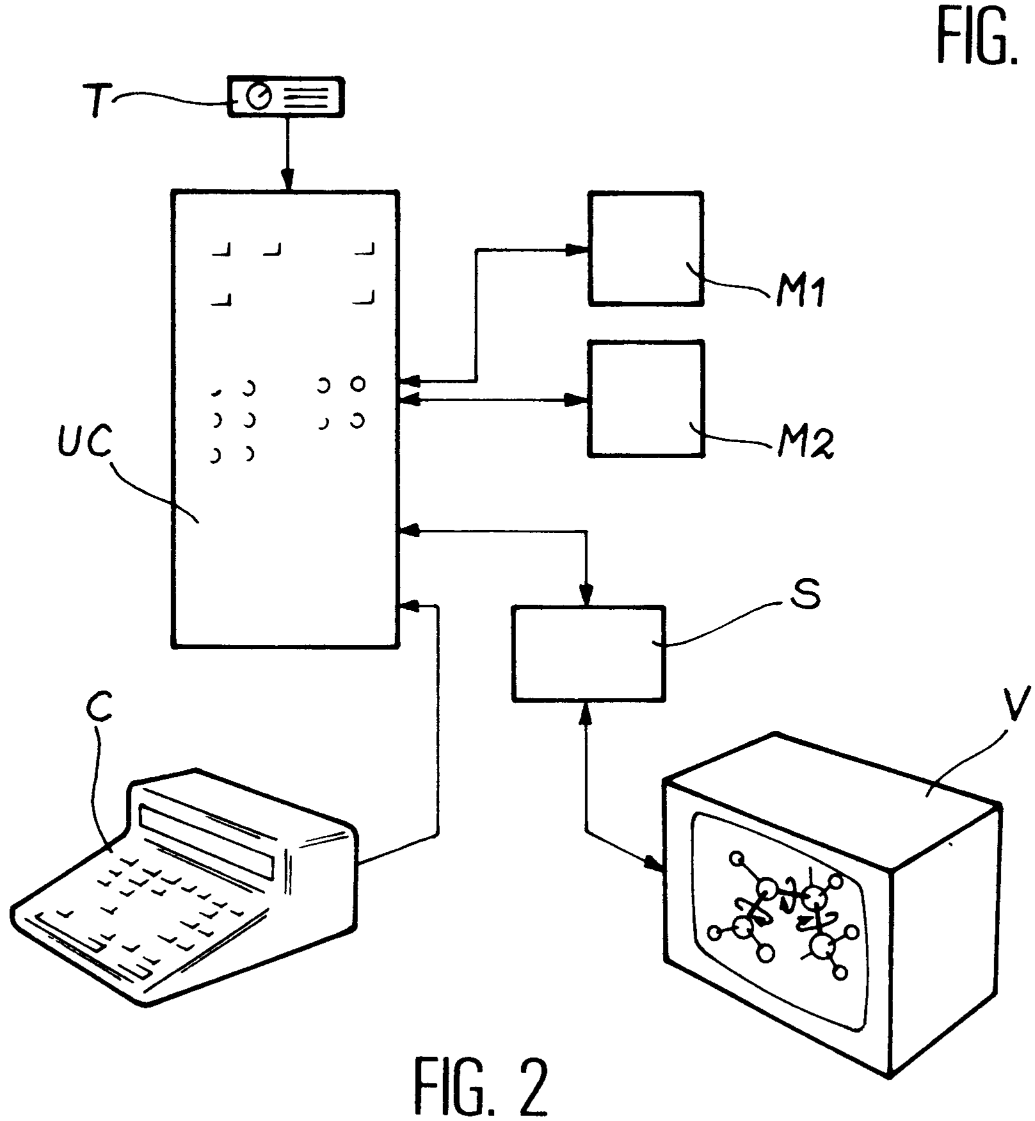


FIG. 2

Office européen des brevets

# RAPPORT DE RECHERCHE EUROPEENNE

Numero de la demande

EP 92 40 0608

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 908 781 (C LEVINTHAL ET AL.) 13 Mars 1990<br>* le document en entier * | 1-11 | G06F15/60<br>C07K3/00 |
| A | CHEMICAL ABSTRACTS, vol. 114, no. 25, 24 Juin 1991, Columbus, Ohio, US;<br>abstract no. 243420P,<br>L H WEAVER ET AL.: 'some uses of the ramachandran (phi, psi) diagram in the structural analysis of lysozymes'<br>page 381 ;<br>* abrégé *<br>& CURR. SCI<br>vol. 59, no. 17, 1990,<br>pages 833 - 837; | 1-11 | |
| A | CHEMICAL ABSTRACTS, vol. 112, no. 1, 1 Janvier 1990, Columbus, Ohio, US;<br>abstract no. 2887Z,<br>J R GAREL ET AL.: 'statistical mchanics model of protein folding: short and long chains have different folding transitions'<br>page 292 ;<br>* abrégé *<br>& J. PHYS.<br>vol. 50, no. 20, 1989, PARIS<br>pages 3067 - 3074; | 1-11 | **DOMAINES TECHNIQUES RECHERCHES (Int. Cl.5)**<br>G06F<br>C07K |
| A | CHEMICAL ABSTRACTS, vol. 109, no. 17, 24 Octobre 1988, Columbus, Ohio, US;<br>abstract no. 144952D,<br>T GAREL ET AL.: 'chemical sequence and spatial structure in simple models of biopolymers'<br>page 305 ;<br>* abrégé *<br>& EUROPHYS. LETT.<br>vol. 6, no. 7, 1988,<br>pages 597 - 601; | 1-11 | |

Le présent rapport a été établi pour toutes les revendications

| Lieu de la recherche | Date d'achèvement de la recherche | Examinateur |
|---|---|---|
| LA HAYE | 18 JUIN 1992 | masturzo |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)